# EUROPEAN PATENT APPLICATION

(11) **EP 3 988 642 A1**
(43) Date of publication of application: **27.04.2022**
(21) Application number: 20202712.4
(22) Date of filing: 20.10.2020
(51) Int. Cl.: C12M 3/06, B01L 3/00, C12M 1/34, G01N 33/487

(54) **SYSTEM FOR MONITORING THREE-DIMENSIONAL CELL CULTURES**

(71) Applicant: Baden-Württemberg Stiftung gGmbH, 70174 Stuttgart (DE)
(72) Inventor: Weltin, Andreas, 79114 Freiburg (DE); Dornhof, Johannes, 79117 Freiburg (DE); Kieninger, Jochen, 79211 Denzlingen (DE); Maurer, Jochen, 52072 Aachen (DE); Urban, Gerald, 79102 Freiburg (DE)
(74) Representative: Müller-Boré & Partner Patentanwälte PartG mbB

(57) **Abstract**

The present invention relates to a system for monitoring three-dimensional cell cultures, an organ-on-chip device comprising the system, and a method for monitoring cell cultures, preferably three-dimensional cell cultures.

## Description

The present invention relates to a system for monitoring three-dimensional cell cultures, an organ-on-chip device comprising the system, and a method for monitoring cell cultures, preferably three-dimensional cell cultures.

In cancer research, in vitro cultured, heterogeneous tumor systems are essential models for the development of personalized chemotherapy. Tumor replicates, generated from stem cells, that are as exact as possible enable research on intercellular processes and interactions, as well as on the efficacy of and resistance to therapy. Due to a large number of replicas of the tumor analyzed in parallel, many pharmacological components can be tested quickly and efficiently. Because of ethical concerns, economical drawbacks and the very limited accessibility of animal models, that is almost impossible in vivo. For this purpose, organ-on-chip systems have been envisaged which try to imitate the in vivo situation and thus obtain a realistic behavior of the cells.

However, the focus for biological applications such as drug screening is on high parallelization, and therefore, comparatively simple readout methods such as fluorescence microscopy are still used. At the same time, elementary aspects of cell culture such as local metabolic parameters or oxygen supply, especially in complex 3D environments, are not known. It would be of great interest to monitor such values by time- and space-resolved measurements, e.g. for the understanding and standardization of basic culture conditions and the investigation of mechanisms of action as well as efficiency of therapeutics and their interaction with the local environment. However, so far, respective means are not available.

Thus, the technical problem underlying the present invention is to provide means for monitoring cells, preferably three-dimensional cell cultures, in a controlled environment and to evaluate by time- and space-resolved measurements the behavior of the cells under defined conditions.

The solution to the above technical problem is achieved by the embodiments characterized in the claims.

In particular, the present invention relates to a system for monitoring three-dimensional cell cultures, wherein the system comprises
a substrate,
an isolation material disposed on the substrate and having openings,
electrodes disposed in the openings of the isolation material, and
a fluidic unit disposed on the isolation material and having a first fluidic channel, a first cell compartment, a second fluidic channel, a second cell compartment, a third fluidic channel in this order along the surface of the isolation material, and barrier structures, which have openings, at each interface between the cell compartments and the fluidic channels;
wherein in at least one of the fluidic channels at least one working electrode is provided and
at least one reference electrode and at least one counter electrode are provided at a second end in fluid direction of at least one of the fluidic channels.

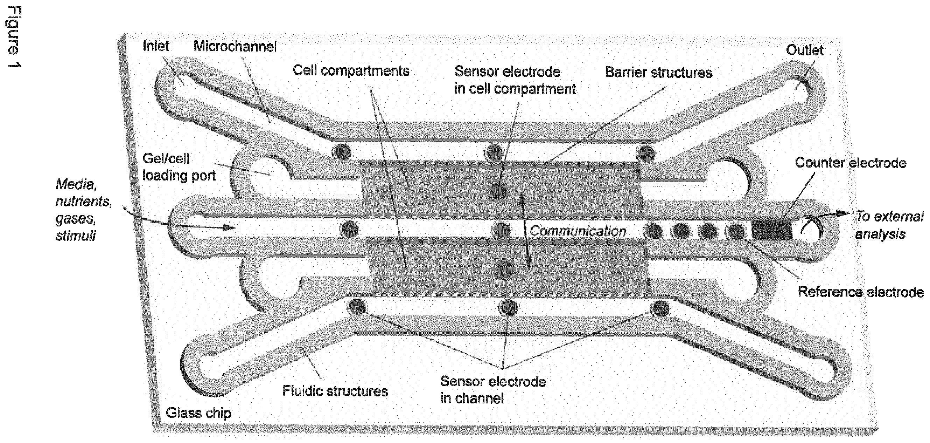

A system is developed that makes it preferably possible to mimic the complexity of a tumor, such as a breast cancer tumor. In this system, organoids consisting of e.g. breast cancer stem cells (BCSC) and tumor-induced fibroblasts are cultivated in an extracellular matrix (Matrigel) and supplied with nutrients or stimuli (e.g. therapeutics) via (micro-)fluidic channels (Fig. 1). In addition, these channels allow for a targeted control of culture conditions and intercellular communication. The system preferably comprises electrochemical microsensors integrated on the chip at the bottom, which allows in-line recording of metabolic parameters such as oxygen, glucose and lactate.

Herein, the term "system for monitoring three-dimensional cell cultures" preferably relates to a sensor system for monitoring three-dimensional cell cultures, more preferably to a sensor system, which is suitable for an organ-on-chip system. The term "organ-on-chip" is preferably used for any chip-based 3D culture model with organotypic functions.

The substrate is not particularly limited. Accordingly, any substrates known in the art can be applied. For example, the substrate can be selected from glass, silicon, ceramics, and polymers. Preferably, the substrate is selected from glass and polymers. More preferably, the substrate is a glass substrate. The glass substrate is preferably a borosilicate glass substrate. Preferably, the substrate is processed in a full-wafer clean room process.

The term "isolation material" relates to a non-conductive material. The isolation material is not particularly limited. Accordingly, any isolation material known in the art can be applied. For example, the isolation material can be selected from silicon nitride, silicon oxide, silicon oxy-nitride, spin-on glass, epoxy-based permanent photoresist, polyimide or dry-film-resist. Preferably, the isolation material is made of silicon nitride and/or silicon oxide.

The isolation material can be applied on the substrate by means commonly known in the art. Suitable methods are for example plasma-enhanced chemical vapor deposition (PECVD), spin-coating and lamination. Preferably, the isolation material is applied by PECVD on the substrate.

The isolation material has openings in which the electrodes can be present. The openings can be generated by suitable means known in the art. For example, the openings can be generated by reactive ion etching, ion beam etching, wet chemical etching, and laser-structuring. Preferably, the openings are generated by reactive ion etching.

The electrodes can be applied directly on the substrate or can be applied on a layer of isolation material applied on the substrate. Preferably, a layer of isolation material is present between the substrate and the electrodes. In case a layer of isolation material is present between the substrate and the electrodes, a first layer of isolation material is applied on the substrate, then the electrodes are applied on the isolation material at the respective intended locations, and then a second layer of isolation material is applied on the first layer of isolation material and the electrodes. The electrodes can then be laid open by generating the openings as described above.

The electrode material can be applied by conventional methods known in the art. Such methods include thin-film technology comprising the evaporation of a metal element, sputtering of a metal element, screen-printing of conductive materials, and etching of a conductive material. For example, there may be evaporated a metal element as base electrode material and optionally another metal element as adhesion layer. Metal elements which can be used as base electrode material are for example platinum, gold, and palladium. A metal element which can be used as adhesion layer material is for example titanium. Preferably, platinum is used as the base electrode material and titanium is used as adhesion layer material. The thickness of the electrode material is not particularly limited and may for example be from 10 nm to 300 nm, preferably from 50 nm to 200 nm, more preferably from 75 nm to 150 nm, and most preferably from 90 nm to 110 µm. A structuring treatment can be carried out at the electrodes, for example by lift-off.

Depending on the purpose of the electrodes, the (laid open) electrodes can be further modified. For example, there may be disposed hydrogels on the electrodes, which can for example be used for diffusion limitation. As the hydrogels, there can e.g. be used poly(2-hydroxyethyl methacrylate) (pHEMA), cross-linked protein matrices, polyacrylamides, Nafion, and chitosan. Preferably, pHEMA is used as the hydrogel. The height of the hydrogel is not particularly limited. For example, the height may be from 1.0 µm to 100 µm, preferably from 10 µm to 50 µm, and more preferably from 20 µm to 40 µm. The hydrogels can for example be dispensed in liquid form onto the electrodes and then cross-linked by UV irradiation. Moreover, in case the electrode is used as a reference electrode, the electrode can for example be modified by electrodeposition of Ag/AgCI on the electrode. In case the electrode is used as a biosensor electrode, the electrode can for example be modified by electrodeposition of a perm-selective membrane on the electrode. Moreover, in case the electrode is used as a biosensor, the respective enzyme is embedded within the hydrogel. In case the electrode is used as a counter electrode, the electrode can be left untreated.

The system according to the present invention further comprises a fluidic unit which is disposed on the isolation material. The fluidic unit has a first fluidic channel, a first cell compartment, a second fluidic channel, a second cell compartment, a third fluidic channel in this order along the surface of the isolation material (thus, not on top of each other with respect to the isolation material), and barrier structures, which have openings, at each interface between the cell compartments and the fluidic channels. The fluidic channels and the cell compartments are arranged alternately with barrier structures in-between, which have openings. The barrier structures having openings can be provided along the whole length of the fluidic channels and the cell compartments (on the sides where the same are in contact with each other) or can only be provided at parts of the fluidic channels and the cell compartments, in particular in parts where the fluidic channels and the cell compartments are in contact with each other. The fluidic channels and the cell compartments are not necessarily in contact with each other along their whole length. For example, the fluidic units may have a central part where the fluidic channels and the cell compartments are in contact with each other in a parallel fashion and may have two end portions at opposite ends (in fluid direction) where the fluidic channels and the cell compartments are separated by walls as barrier structures having no openings (to the side). Such walls are also provided at the outer circumference of the fluidic unit, such as at the ends of the cell compartments and the fluidic channels and at the outer sides of the most outward fluidic channels (cf. Fig. 1). The walls can be made of the same material as the fluidic channels and the cell compartments.

The fluidic channels and the cell compartments preferably each have two end portions at opposite ends, wherein at the first end the fluid and the cell(s), respectively, are inserted in the fluidic channels and the cell compartments, respectively. At the second end of the fluidic channels, there are preferably provided outlets, where the fluids can exit the system. Thus, the fluidic channels are preferably configured to provide a fluid flow from one end of the fluidic channel to the other end of the fluidic channel. The cell compartments are preferably configured to receive cells in a liquid matrix. The matrix then solidifies to yield cells embedded in a solid matrix in the cell compartments.

Techniques for disposing the fluidic channels, the cell compartments, and the barrier structures are known in the art. Exemplary techniques include lithography, such as photolithography or depth lithography, thin-film deposition, etching techniques, such as wet etching or dry etching, casting techniques, e.g. soft lithography, injection molding, bonding techniques including silicon direct bonding, anodic bonding or glass bonding, plasma-activated bonding or maskless patterning techniques such as laser micromachining, inkjet printing or 3D printing. Examples of suitable materials for the fabrication of the fluidic unit include, for instance, epoxy-based permanent photoresists, epoxy-based dry-film-resists, glass, additively manufactured glass, thermoplastic polymers, polymers for additive manufacturing, polymer-based dry-film-resists and silicone. Preferably, the material for the fluidic unit is selected from permanent epoxy photoresists, epoxy-based dry-film-resists, and dry-film-resists. Most preferably, the material for the fluidic unit is the permanent epoxy photoresist SU-8 3000.

In a preferred embodiment, the fluidic unit is manufactured by means of photolithography, such as an epoxy-based permanent photoresist (SU-8) technology. In this case, the fabrication of the fluidic unit may be realized on wafer-level, using a substrate as indicated above and photolithographic techniques, employing multiple SU-8 layers.

In a preferred embodiment, the width of each of the cell compartments is from 100 µm to 10 mm, more preferably from 250 µm to 5.0 mm, more preferably from 500 µm to 2.0 mm, most preferably 1.0 mm.

Preferably, the width of each of the fluidic channels is from 20 µm to 2.0 mm, more preferably from 40 µm to 1.0 mm, most preferably 500 µm.

The cross-sectional shapes of the inner surface of the fluidic channels and the cell compartments are not particularly limited. Preferably, the fluidic channels and the cell compartments have an inner surface with a rectangular shaped cross-section.

In a preferred embodiment of the invention, the length of each of the cell compartments is from 100 µm to 100 mm, preferably from 1.0 mm to 10 mm, most preferably from 3.0 mm to 6.0 mm.

Preferably, the length of each of the fluidic channels is from 200 µm to 100 mm, preferably from 1.0 mm to 50 mm, most preferably from 4.0 mm to 15 mm.

In a preferred embodiment of the invention, the height of each of the cell compartments is from 20 µm to 2.0 mm, preferably from 50 µm to 1.0 mm, most preferably from 100 µm to 500 µm.

Preferably, the height of each of the fluidic channels is from 20 µm to 2.0 mm, preferably from 50 µm to 1.0 mm, most preferably from 100 µm to 500 µm.

The dimensions of the barrier structures preferably partially correspond to the dimensions, preferably the height and the length, of the cell compartments and the fluidic channels. In a preferred embodiment, the length of each of the barrier structures having openings is from 100 µm to 100 mm, preferably from 1.0 mm to 10 mm, most preferably from 3.0 mm to 6.0 mm. The height of each of the barrier structures having openings (highest part of the barrier structures) is from 1.0 µm to 10.0 mm, preferably from 10 µm to 1.0 mm, most preferably from 100 µm to 500 µm. Preferably, the width of each of the barrier structures having openings is from 50 µm to 500 µm, more preferably from 75 µm to 150 µm, most preferably 100 µm.

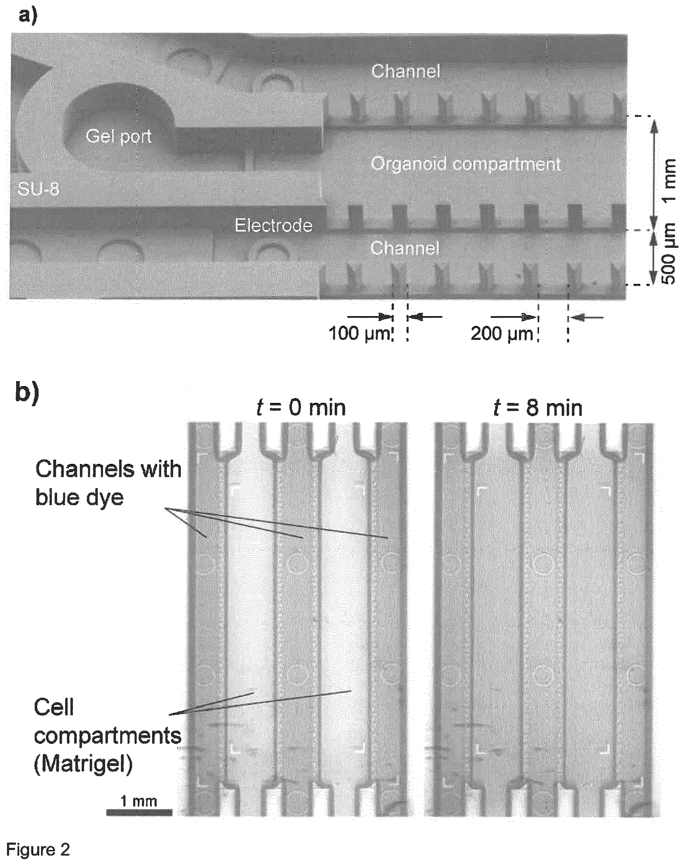

In a preferred embodiment, the barrier structure comprises a continuous flat step and a plurality of posts provided on the step in equal distances along the fluid flow. The continuous flat step has the same width and length as the barrier structure. The step preferably has a height of from 1.0 µm to 200 µm, more preferably from 10 µm to 100 µm, more preferably from 20 µm to 80 µm, most preferably from 30 µm to 60 µm. The distance between two adjacent posts may for example be from 10 µm to 1.0 mm, preferably from 50 µm to 500 µm, more preferably from 100 µm to 300 µm, most preferably from 150 µm to 250 µm. The posts can have different cross-sectional shapes (when viewed from the top of the system, i.e. from the height direction of the barrier structures), such as triangular, trapezoid, hexagonal, and half-circular shapes. Preferably the posts have a triangular, half-circular or trapezoid shape. More preferably, the posts have a triangular, more preferably an equilateral triangular, cross-sectional shape. The sides of the triangles may have a length of from 10 µm to 500 µm, preferably from 30 µm to 300 µm, more preferably from 50 µm to 200 µm, most preferably from 80 µm to 120 µm. The triangles preferably have a flat side pointing to the side of the fluidic channels and a vertex pointing to the side of the cell compartments (cf. Fig. 2a).

With the help of the barrier structures having openings it is preferably possible to fill the cell compartments with a matrix, such as Matrigel, in any concentration in a robust manner such that the matrix is prevented from leaking into the fluidic channels. Such filling process can preferably be carried out with a standard laboratory pipette directly into the system and can preferably also be carried out by untrained users, as the barrier structures preferably guarantee controlled filling. At the same time, the barrier structures having openings preferably enable the diffusion of molecules into and out of the cell compartments. Fluidic channels filled with dyed medium (blue) and the diffusion of the dye into cell compartments filled with matrix gel (red) are shown in Fig. 2b.

According to the present invention, in at least one, preferably in each, of the fluidic channels at least one working electrode is provided and at least one reference electrode and at least one counter electrode are provided at the second end (in fluid direction) of at least one of the fluidic channels. Furthermore, preferably at least two, more preferably at least three, working electrodes are provided in each of the fluidic channels. Preferably, the reference electrode(s) and the counter electrode(s) are provided in the second fluidic channel. Moreover, in the second fluidic channel, there are preferably provided at least four, more preferably at least five, working electrodes. Furthermore, at least one working electrode may be provided in at least one, preferably in each, of the cell compartments.

In case one working electrode is provided in a fluidic channel, said working electrode is preferably provided in the middle of the fluidic channel (in fluid direction). In case more than one working electrode is provided in a fluidic channel, the further working electrode(s) is/are preferably provided at the first end (in fluid direction) of the respective fluidic channel, e.g. shortly before (in fluid direction) the beginning of the barrier structure, and/or at the second end (in fluid direction) of the respective fluidic channel, e.g. shortly after (in fluid direction) the end of the barrier structure. In case one working electrode is provided in a cell compartment, said working electrode is preferably provided in the middle of the cell compartment (in fluid direction) and in the center between the barrier structures.

The reference electrode(s) is/are preferably provided at the second end (in fluid direction) of the respective fluidic channel after all working electrodes. The counterelectrode(s) is/are preferably provided at the second end (in fluid direction) of the respective fluidic channel after all working electrodes. Moreover, the reference electrode(s) is/are preferably provided closer to the center of the respective fluidic channel than the counter-electrode(s). Preferably, a reference and a counter electrode are provided in each fluidic channel.

The electrodes can have different shapes such as circular, square, rectangular, oval, and comb-like shapes. Preferably, counter-electrodes have a rectangular shape. In a preferred embodiment, working electrodes and reference electrodes have circular shapes.

The working electrodes can be used for monitoring different substances/conditions, such as oxygen, lactate, glucose, glutamate, choline, pyruvate, superoxide, nitric oxide, dopamine, hydrogen peroxide, and pH. For monitoring lactate and/or glucose, hydrogels are preferably disposed on the electrode material as described above and enzymes are included in the hydrogels, which convert lactate and glucose, respectively, to a substance, such as hydrogen peroxide, which can be detected at the electrode. For monitoring oxygen, the electrodes can be used without further modification.

Working electrodes for monitoring oxygen (oxygen microsensors) preferably have a circular shape. The diameter of the oxygen microsensors is not particularly limited. For example, the diameter may be from 5.0 µm to 500 µm, preferably 50 to 400 µm, more preferably 100 to 300 µm, most preferably 200 µm. In a preferred embodiment, at least one electrode for monitoring oxygen is provided in at least one, preferably in each, of the fluidic channels. Preferably, at least three electrodes for monitoring oxygen are provided in any, preferably the second, fluidic channel, more preferably in the cell compartments and the second fluidic channel, and most preferably in each of the cell compartments and the fluidic channels.

Working electrodes for monitoring glucose and/or lactate (biosensors for glucose and/or lactate) preferably have a circular shape. The diameter of the biosensors for glucose and/or lactate is not particularly limited. For example, the diameter may be from 50 µm to 800 µm, preferably from 100 µm to 600 µm, more preferably from 200 µm to 400 µm, most preferably 300 µm. In a preferred embodiment, an electrode for monitoring glucose and/or an electrode for monitoring lactate is provided at one end of any, preferably the second, fluidic channel.

The diameter of reference electrodes is not particularly limited. For example, the diameter may be from 10 µm to 600 µm, preferably from 50 µm to 500 µm, more preferably from 100 µm to 400 µm, most preferably 300 µm.

The dimensions of the counter electrodes are not particularly limited. Preferably, the area is 0.1 mm² to 10 mm², more preferably 0.2 mm² to 1.0 mm², most preferably 0.3 mm² to 0.5 mm².

For protecting the electrodes and/or the modifications of the electrodes, there can be disposed the same material as used for the cell compartments and the fluidic channels around the electrodes, e.g. on the isolation material at the border of the openings for the electrodes. For example, the height of this (protective) structure may be from 1.0 µm to 200 µm, preferably from 10 µm to 100 µm, most preferably from 30 µm to 50 µm. Its shape and dimension follow that of the respective electrode. The distance between electrode edge and this (protective) structure inner edge may be from 1.0 µm to 100 µm, preferably from 10 µm to 50 µm. The distance from the inner to the outer edge of the (protective) structure may be from 5.0 µm to 1.0 mm, preferably from 10 µm to 100 µm, most preferably from 20 µm to 50 µm.

The system may further comprise connecting means, which can be used for connecting the electrodes to means for detecting the signals of the electrodes. Such connecting means are known in the art. Examples of such connecting means are circuit paths and contact pads. For example, individual electrodes can be connected via (non-intersecting) circuit paths to individual contact pads, which are situated on the surface of the substrate outside the area where the fluidic unit is formed. Contact pads can then be used for establishing a connection to means for detecting the signals of the electrodes. The contact pads and circuit paths can be formed and connected with the electrodes in the same step and with similar materials as described for the formation of the electrodes above.

Preferably, the system further comprises a cover having openings corresponding to each of the cell compartments and the fluidic channels. The openings can be used for inserting the cell(s) with the matrix at the first ends of the cell compartments. Moreover, the openings can be used for inserting fluids at the first ends of the fluidic channels and for outlets of fluids at the second ends of the fluidic channels. Preferably, the cover is made of a transparent material. With such a transparent material it is possible to monitor the system also by optical methods. Moreover, the cover preferably closes the system in a gas-tight manner. The cover is preferably permanently fixed to the fluidic unit by underfilling with an adhesive. Preferably, this adhesive is an UV-curable adhesive or two component epoxy. The edges of the fluidic unit serve to define the distribution of the adhesive by capillary forces. The attachment preferably occurs at room temperature so that temperature sensitive enzymes can be included in the process. For further sealing during operation, the openings for loading of the cell compartments can be sealed, e.g. with polyimide-based adhesive tape.

The material of the cover is not particularly limited. For example, PMMA, polystyrene, glass, COC, polycarbonate, or PDMS can be used as material for the cover. Most preferably, the cover is made of PMMA.

The fluidic structures, aside from the continuous flat step, are designed such that they can also be directly integrated with the cover. In this case, the cover including the fluidic structures may be fabricated in one step. For example, this fabrication can be done by injection molding or thermoforming of polymers or by additive manufacturing of both polymers and glass.

Moreover, the present invention relates to an organ-on-chip device comprising the system according to the present invention, an electrical interface to which the system is bonded, and cell cultures, preferably three-dimensional cell cultures, embedded in the cell compartments of the system. The above definitions analogously apply to this aspect of the present invention. Preferably, the cell cultures are embedded in a matrix in the cell compartments of the system.

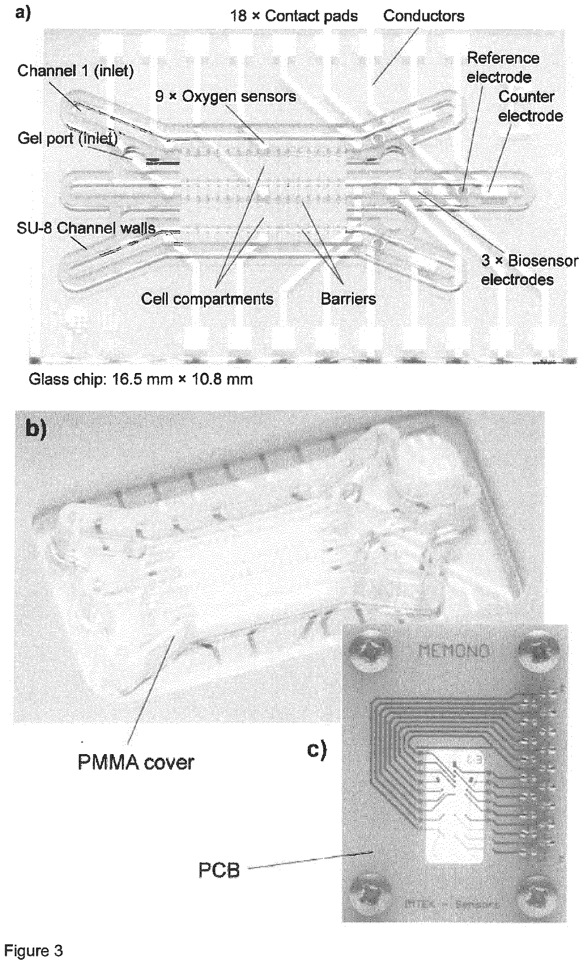

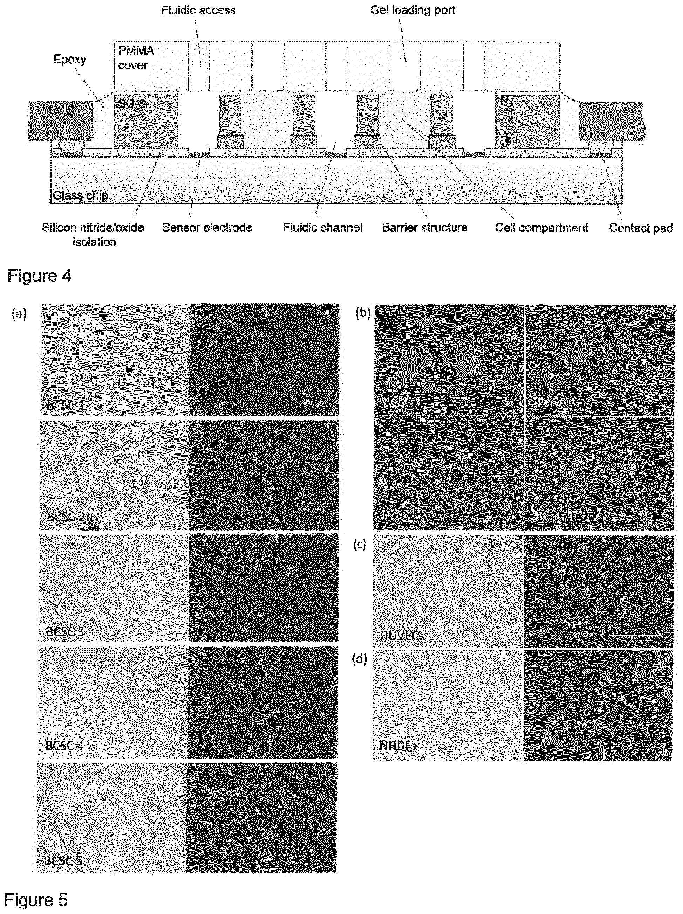

Means for assembling and electrically interfacing the system according to the present invention are known in the art. For example, the system can be flip-chip bonded to an electrical interface or a circuit board, particularly a printed circuit board (cf. Figs. 3 and 4). The electrodes of the system according to the present invention can be connected to the electrical interface e.g. by connections means as mentioned above.

Suitable matrix materials are known in the art. For example, there can be used Matrigel, Geltrex Matrix Products, alginates, agarose, hydrogels from Cellendes, HyStem, Xylyx or component mixtures of vitronectin, fibronectin, laminin and collagens as the matrix material. Preferably, Matrigel is used as the matrix material. The concentration of the cells in the matrix is not particularly limited. For example, the concentration of the cells in the matrix is a single cell to 100000000 cells/ml, preferably 10000 cells/ml to 10000000 cells/ml, more preferably 50000 cells/ml to 5000000 cells/ml, and most preferably 100000 cells/ml to 2000000 cells/ml.

As the cells, there can in principle be introduced any cells of interest. For example, there can be introduced tumor cells, stromal cells, endothelial cells, normal stem cells, cultivated and tissue isolated cell material from any human or animal organ source, preferably triple negative human breast cancer stem cells.

The mixtures of cells in matrix are preferably introduced in the cell compartments at a temperature at which the matrix is in a liquid state. After the introduction of the mixture, the matrix solidifies at elevated temperature to yield the cells embedded in the matrix in the cell compartments.

Moreover, the present invention relates to a method for monitoring cell cultures, preferably three-dimensional cell cultures, wherein the method comprises the steps of providing an organ-on-chip device according to the present invention, introducing one or more fluids to one or more fluidic channels, and
detecting the signals of the electrodes. The above definitions analogously apply to this aspect of the present invention.

Means for detecting the signals of electrodes are known in the art. For example, the signals can be detected by amperometry, chronoamperometry, cyclic voltammetry, differential pulse voltammetry, square wave voltammetry, fast scan cyclic voltammetry, potentiometry and active potentiometry for chemical sensors (e.g. oxygen) and biosensors (e.g. lactate).

The fluids which are introduced in the fluidic channels are not particularly limited. The fluids can contain different kinds of nutrients, stimuli, and enclosed gases. As stimuli, there can for example be introduced drugs, growth factors, and nutrients. The fluids can be varied over time and can differ in each fluidic channel, so as to monitor the different behavior of the cells to the different types of fluids.

The types of fluids may be cell culture media or aqueous buffer solutions, which are known in the art. The fluids may contain a defined chloride ion concentration, preferably between 0.05 molar and 0.15 molar. The flow rates at which fluids are supplied may be from 0.1 µl/min to 100 µl/min, preferably from 1 µl/min to 20 µl/min.

The figures show:
Fig. 1: Top view of the system (without cover) having two gel-filled organoid/cell compartments and adjacent medium/fluidic channels. Electrochemical microsensors are integrated at the bottom of the platform and allow real-time monitoring of oxygen, glucose and lactate.
Fig. 2: a) Scanning electron microscope image of the barrier structures that separate the fluidic channels (width = 500 µm) from the organoid compartments (1 mm x 5 mm). b) Diffusion of blue dye from the channels into the Matrigel.
Fig. 3: a) Sensor chip with fluidic structures and microelectrodes, b) Encapsulated system with PMMA cover, c) Assembled system with PCB (printed circuit board).
Fig. 4: Technological cross-section of an organ-on-chip system with sensor chip, fluidic unit, electrical connection, and cover.
Fig. 5: Microscopic images of fluorescence-transduced cells: a) Green fluorescence staining of the 5 BCSC cell lines, (left - phase contrast (10X), right - corresponding GFP image), b) Autofluorescence images of 4 BCSC cell lines, c) Human Umbilical Vein Endothelial Cells (HUVEC), (Left - phase contrast (10X), Right - corresponding GFP image), d) Neonatal Human Dermal Fibroblast Cells (NHDF), (Left - phase contrast (10X), Right - corresponding RFP image).

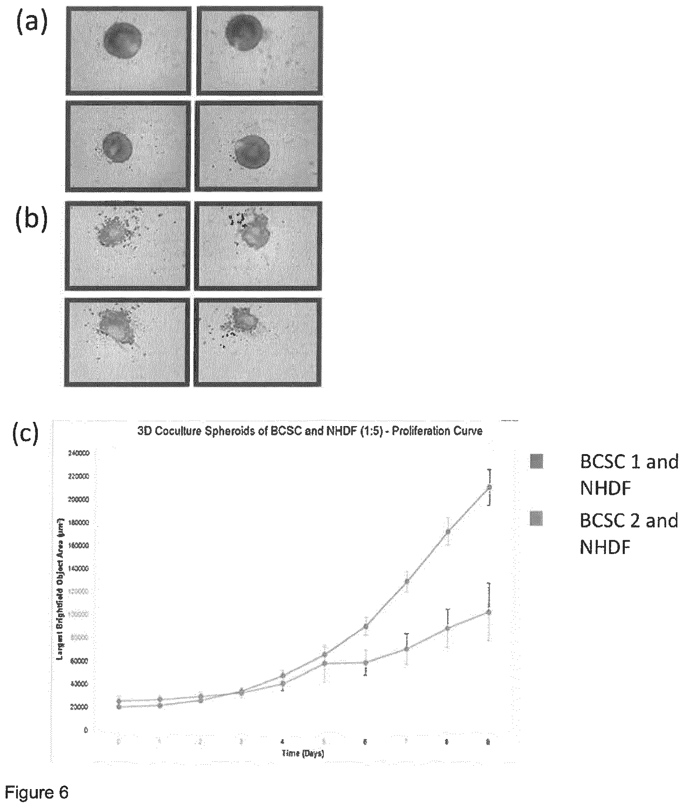

Fig. 6: Creation of reproducible single organoids and observation of cell growth using high-throughput live cell imaging (Incucyte), a) 3D co-culture of BCSC 1 (unlabelled) and NHDFs (RFP) in a ratio of 1:5, b) 3D co-culture of BCSC 2 (unlabelled) and NHDFs (RFP) in a ratio of 1:5, c) growth curve of 3D co-culture organoids of BCSC1, BCSC 2 with NHDFs in a ratio of 1:5.

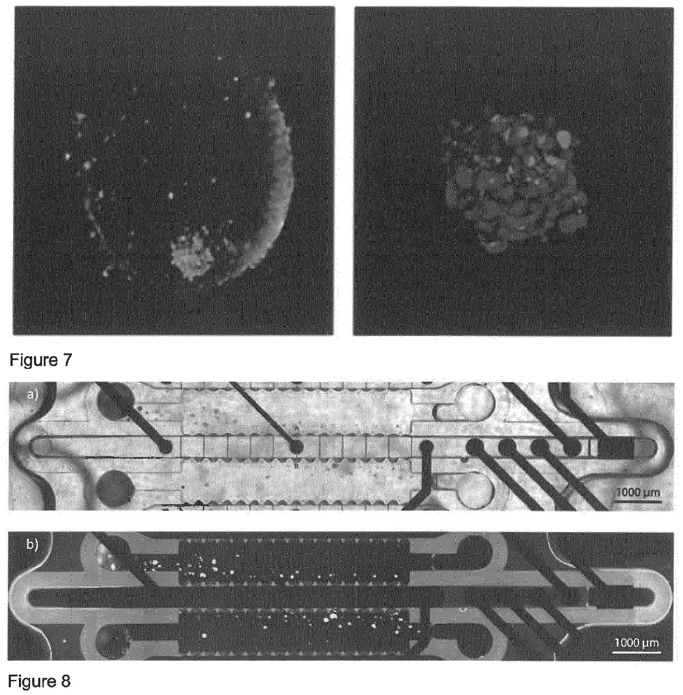

Fig. 7: 2-photon microscopy images of BCSC 3D co-culture with NDHF, taken after 13 days to show the integration of both cell types in one organoid (a) 3D co-culture of BCSC 1 (GFP) and NHDF (RFP) in a 1:5 ratio, (b) 3D co-culture of BCSC 2 (autofluorescent) and NHDF (RFP) in a 1:5 ratio.
Fig. 8: Phase contrast image (a) and GFP fluorescence image (b) of seven days old BCSC organoids (Ø <100 µm) embedded in 50 % matrices and filled into the two cell compartments of the system.

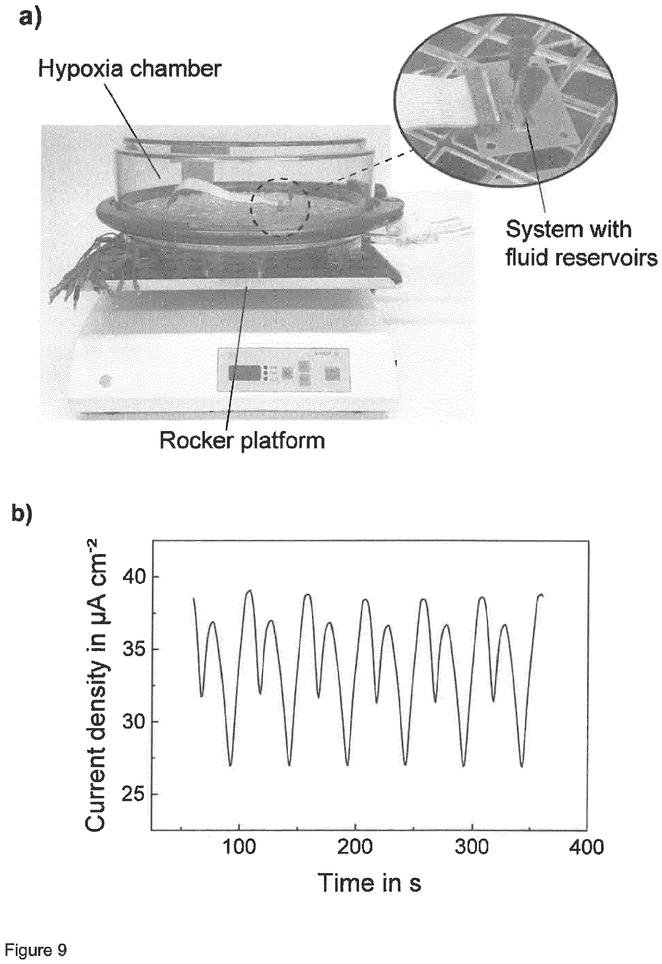

Fig. 9: a) Experimental setup for gravitationally driven media circulation. b) Sensor signal of an oxygen sensor located in the fluidic channel (without diffusion-limiting membrane), which then corresponds to the flow rate. The tilting angle during the experiment was ± 12° at a frequency of 1 rpm.

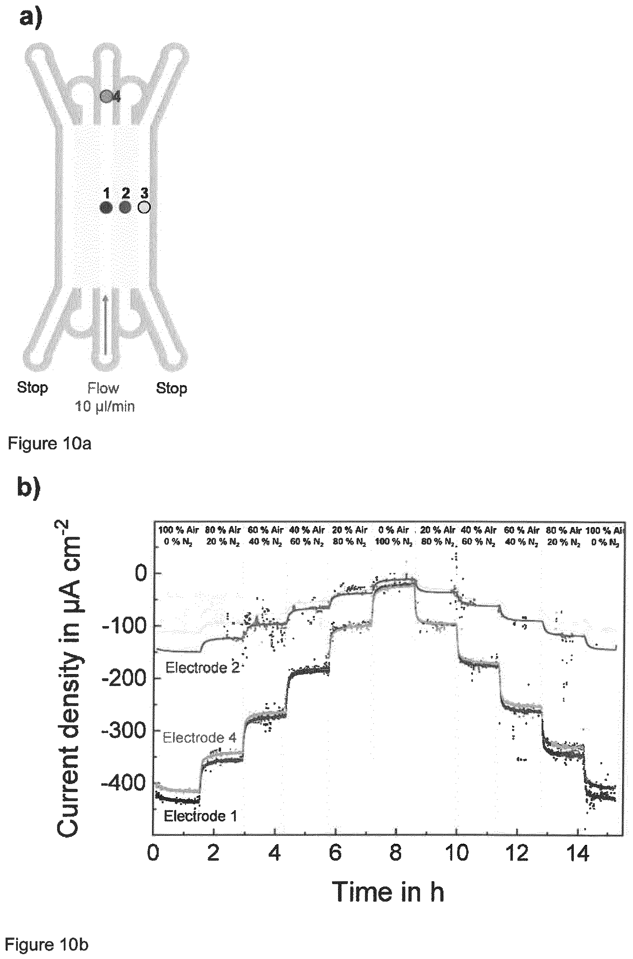

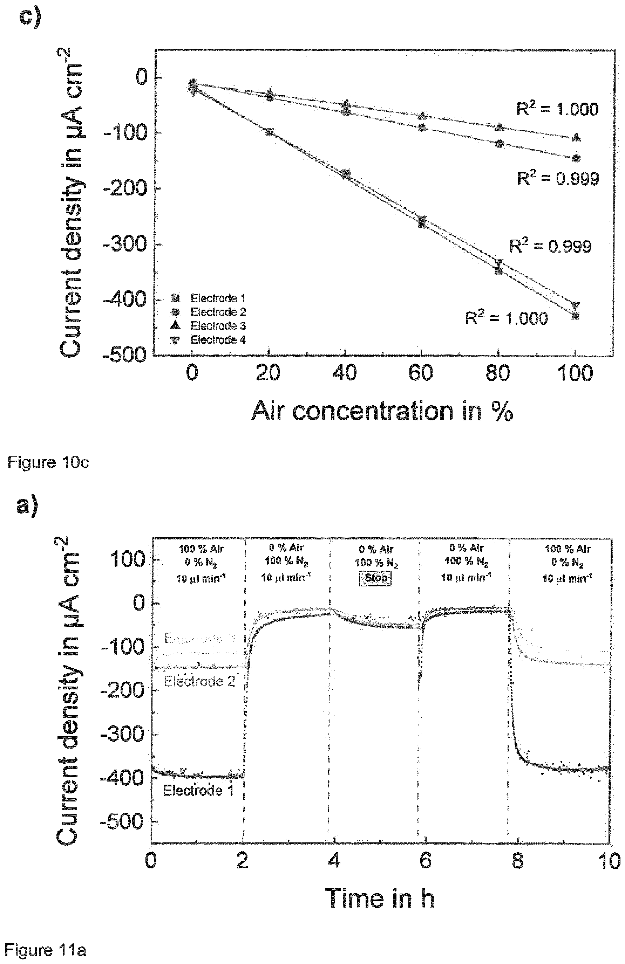

Fig. 10: a) Electrode configuration for oxygen measurements. Two electrodes are located in the middle fluidic channel (electrodes 1 and 4), one in the cell compartment (electrode 2) and one in the fluidic channel behind the cell compartment (electrode 3). The middle channel is operated with 10 µl/min. b) Transient oxygen calibration of the four electrodes with negative reduction current c) Corresponding calibration curves of the four electrodes. A stable, reversible, extremely linear sensor behavior with defined zero point is shown. The magnitude of the sensor signal represents the respective mass transport to the electrode.

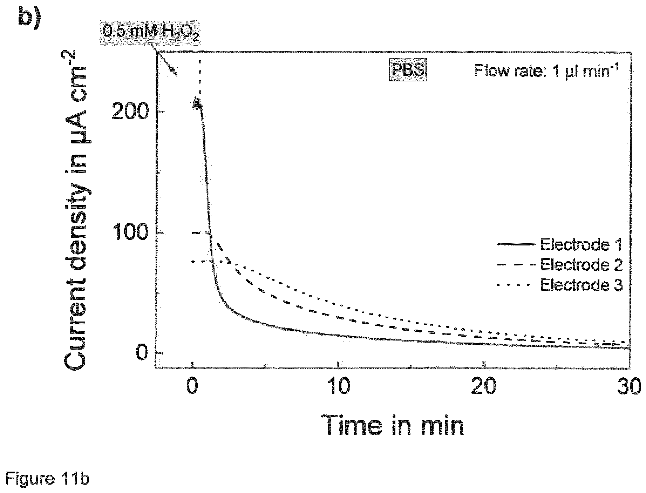

Fig. 11: a) Oxygen measurement (via negative reduction current), in which the flow is stopped with nitrogen-saturated medium. The small signal increase in the stop phase demonstrates the gas tightness of the system; b) Measurement of hydrogen peroxide (via positive oxidation current), which is flushed out of the system. While in the flow channel (electrode 1) a fast change is achieved, the cell compartments release the substance slowly (electrodes 2 and 3), which allows to estimate the dynamics of nutrients and therapeutics in the organ-on-chip system.

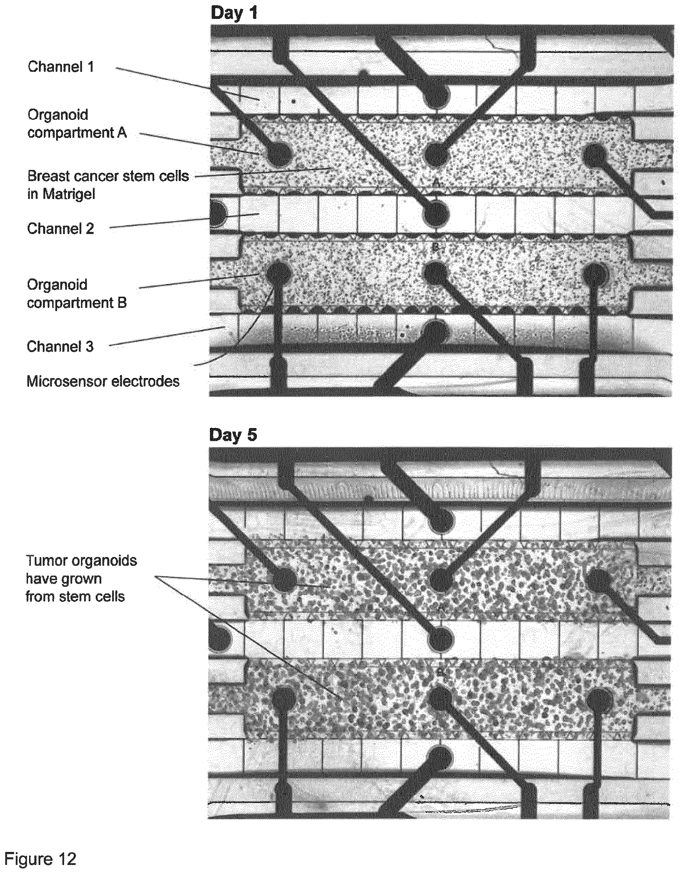

Fig. 12: Culturing of organoid cultures in the system according to the present invention (phase contrast images). Single breast cancer stem cells in Matrigel are seeded into the cell compartments. Over 5 days of culture under microfluidic operation, tumor organoids develop within the system. Cell viability is confirmed by phase contrast and fluorescence microscopy.

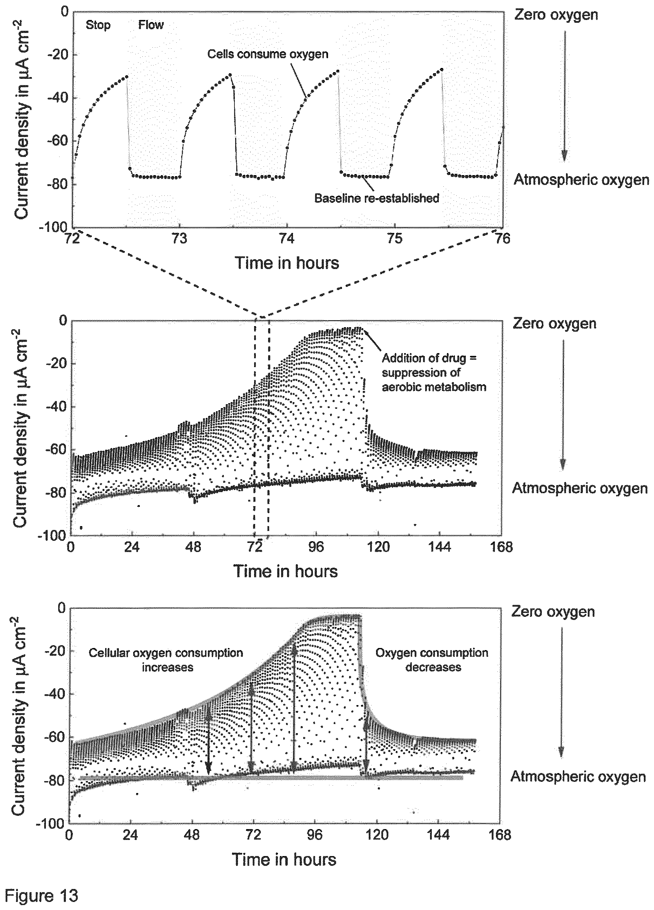

Fig. 13: Monitoring of organoid metabolism. Top: In the stop/flow cycle, cells consume oxygen during the stop phase, and the atmospheric baseline is re-established in the flow phase. Middle/Bottom: Over days, the cellular oxygen consumption increases until almost zero oxygen is reached in the stop phase. A drug to supress aerobic metabolism is added, and oxygen consumption decreases. Cell survival until the end of the experiment is confirmed by phase contrast and fluorescence microscopy.

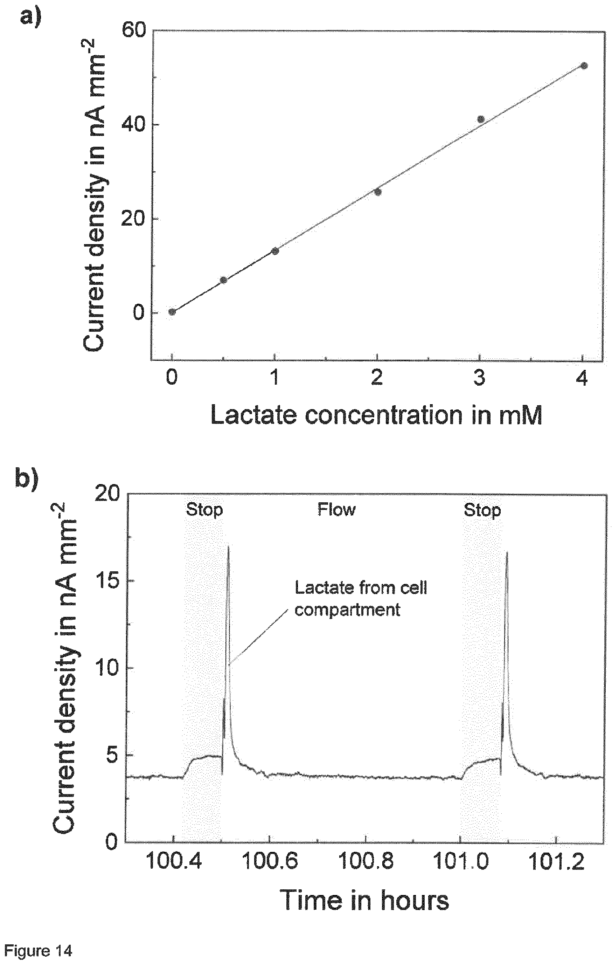

Fig. 14: a) Lactate calibration in cell culture medium. b) Stop/flow cycle of lactate measurement. During the stop phase, cells produce lactate. During the flow phase, this lactate is transported from the cell compartment to the downstream lactate sensor, and a distinct peak is detected. The baseline is re-established after the lactate-rich medium exits the system.

The present invention will be further illustrated in the following examples without being limited thereto.

### Example 1: Fabrication of an organ-on-chip platform

Fabrication of the organ-on-chip platforms starts on wafer-level with a 500 µm thick Pyrex substrate and the deposition of platinum electrodes via thin-film technology comprising the evaporation of platinum as electrode material and titanium as adhesion layer. This is followed by plasma-enhanced chemical vapor deposition (PECVD) of silicon nitride/oxide. The circular shaped (Ø 200 and 300 µm) electrodes are then reopened by means of reactive-ion etching. The fluidic unit including the barrier structures, consists of the negative resist SU-8. For this purpose, multiple layers of it are deposited and structured using two different chrome masks. Thereby, the total amount of layers defines the final thickness of the fluidic unit. In this example, a fluidic unit with a height of around 300 µm was produced (Fig. 3a). In addition, silver/silver chloride is deposited onto one electrode in each channel, which then serves as reference electrode during electrochemical measurement. In the case of counter electrodes, the platinum is left untreated. A further modification of the electrodes takes place via the deposition of hydrogels. For oxygen sensors, the approximately 30 µm thick hydrogel layer acts to limit diffusion. In the case of lactate biosensors, the corresponding enzyme (lactate oxidase) is immobilized in the hydrogel. This enzyme converts the reactants into a by-product and the electrochemically active hydrogen peroxide. Measurement of the hydrogen peroxide concentration then provides indirect insight into the reactant concentration.

After dicing, electrical connection to external read-out electronics (potentiostat) is ensured by means of flip-chip bonding on a printed circuit board (PCB; Fig. 3c and 4). The cover of the chips consists of milled poly(methyl methacrylate) (PMMA; cf. Fig. 3b) and is attached firmly to the fluidic unit with a biocompatible adhesive. Cancer cells can then be filled into the assembled chips at 0 °C in liquid ECM (extra cellular matrix). Subsequent increase in temperature to 37 °C for 30 minutes leads to solidification of the ECM.

### Example 2: Cultivation of different cells lines

The following cell lines were cultivated and fully characterized:
- Different lines of breast cancer stem cells (BCSC) (direct isolates of primary breast cancer patients)
- Neonatal dermal human fibroblasts (NHDF) (Global Stem) and primary fibroblasts from tumor tissue of patients (7 individual cultures)
- Human endothelial cells isolated from cord blood (HUVEC) (Lonza)

The cell systems used were immunofluorescently labelled (GFP, green fluorescence protein) using complex lentiviral transduction (Fig. 5). In addition, the cells were used in different proportions after labelling in order to generate organoid images as close as possible to the tumor (Fig. 6a and b). The growth (expansion of the organoids) and proliferation of the individual cells in the organoid was monitored (Fig. 6c), by an Incucyte system. Finally, the tumor organoids generated in this way were further processed under the microscope using 2-photon microscopy to determine the exact localization and growth of the cells (cf. for example Fig. 7).

### Example 3: Fluidic concepts with cells

First, the filling with cell-loaded matrices of different gel concentrations was investigated. On the one hand, single cells (BCSC) were integrated into the system and, on the other hand, in vitro cultivated BCSC organoids were successfully transferred into the chip using a protocol (cf. Fig. 9).

In the first case, single cells of a 2D cell culture, cultivated outside the platform, were separated using accutase. After cell counting, the desired amount of cells was suspended in a mixture of cell culture medium and Matrigel (ratio 1:3, 75% Matrigel) on ice. In the shown example, 80000 cells were suspended in 40 µl of the mixture. 3.5 µl of the cell/matrix suspension were filled into the platform via the inlet of each cell compartment using a 10 µl pipette equipped with a corresponding pipette tip.

In the second case, seven days old BCSC organoids of a 3D cell culture, cultivated outside the platform, were isolated using dispase. Before counting, organoids were filtered through a 100 µm mesh. The desired amount of organoids was suspended in a mixture of cell culture medium and Matrigel (ratio 1:1). In the shown example 200 organoids were suspended in 20 µl of the mixture. 3.5 µl of the suspension was filled into each cell compartment.

As described above, the newly developed cell systems are lentivirally fluorescently labelled (GFP). This avoids time-consuming and costly external staining methods and allows to microscope cells in a high-throughput process. The phase contrast and fluorescence microscopy images show that a complete optical transparency of the organ-on-chip system is an advantageous feature and that it can be seamlessly integrated into cell biological workflows. A passive pumping principle as described below was used for the fluidic supply of cells under hypoxia during culture.

### Example 4: System integration of fluidics, sensors and electrical connection

The electrical contact of the system was made by flip-chip bonding to a printed circuit board (cf. Fig. 3b,c). Electrical contacts were hermetically sealed to protect them from moisture in the incubator. For integration into a hypoxia box (cf. Fig. 9a), the electrical connection is made via gas-tight plugs to the outside. CompactStat (Ivium Technologies) and MultiEmStat3 (PalmSens) potentiostats were used as instrumentation for the electrochemical sensors. These offer both the possibility to use complex electrochemical measurement methods and continuous monitoring with several channels.

Especially for the culture phase of the cells, passive fluidics without external pumps can be advantageous. In addition to the simplified design, this allows easy parallelization. For this purpose, the fluid circulation was investigated with a passive, gravitationally driven pumping unit. The platform including the hypoxia box, in which the desired gas atmosphere is adjusted by means of a gas mixing station, is placed on a laboratory shaker (cf. Fig. 9a). The tilting movement of the shaker causes a difference in height of the medium reservoirs on the chip. The hydrostatic pressure difference then leads to a media flow (Fig. 9b). With the aid of oxygen sensors, whose signal depends on the mass flow and thus on the flow velocity without a diffusion-limiting membrane, the generated flow rates can be measured in situ. It was observed that a continuous media exchange in the system is possible with this method.

### Example 5: Fluidics and sensors

The electrochemical oxygen sensors, in combination with microfluidics, in the organ-on-chip system were characterized in detail. By means of a gas mixing station, media were gassed with air/nitrogen/carbon dioxide mixtures and pumped through the system. Chronoamperometric protocols were used for oxygen measurement, whereby the oxygen is reduced amperometrically at the electrode.

With the integrated microsensors, it was possible to precisely measure directly in the system and continuously monitor the local oxygen concentrations. Electrodes in the fluidic channel, in the cell compartments and in the fluidic channels behind the cell compartments were investigated (Fig. 10a). Fig. 10b shows a transient oxygen measurement over the entire atmospheric range. Since oxygen is measured by the reduction current, the oxygen concentration is proportional to the measured negative current. The resulting calibrations (Fig. 10c) show a precise, very linear sensor characteristic with a defined zero point without significant offset.

The successful establishment of very low oxygen values in the system also proved the gas tightness of the system. Even when the flow of oxygen-free medium is stopped for 2 hours (Fig. 11a), very little oxygen enters the system from outside. Thus, the system, which is in a normal atmosphere, can be set very precisely and arbitrarily in both normoxic and strongly hypoxic states via the medium alone.

Furthermore, measurements were carried out with hydrogen peroxide, which was oxidized at the oxygen sensors and serves as a test substance that does not occur in the environment. This was used to investigate the mass transport of small molecules in the system, which can later be used for experiments with nutrients or pharmaceuticals.

It was confirmed that microfluidics allow an efficient exchange of concentrations, as expected (Fig. 11b). The cell compartments represent a kind of reservoir that slowly fills with analytes and slowly releases them again. At a very low flow rate of 1 µl/min, a complete exchange of the concentration (t_{90%-10%}) can be achieved in the central fluidic channel within about 5 min. In the center of a cell compartment this value is about 23 min and in the fluidic channel (without flow) behind a cell compartment chamber it is 31 min. The total used media volume remains very low (< 50 µl).

### Example 6: Organoid culture

Breast cancer stem cells are seeded as single cells in Matrigel into the two organoid/cell compartments of the system of the present invention. After filling and solidification of the cell/Matrigel suspension, fluidic ports get sealed with polyimide-based adhesive tape. Channels through which media flow is to be generated are connected to gas-tight tubing. A flow rate of 2 µl/min is applied with a syringe pump. Under microfluidic operation by pumping medium periodically through the channels, tumor organoids develop over days. Oxygen and nutrients are supplied by the microfluidics. Organoid viability is confirmed throughout by phase contrast and fluorescence microscopy (cf. Figure 12)

### Example 7: Organoid Metabolism Monitoring

Breast cancer stem cells (BCSC), as described in Example 2, are seeded as single cells and cultured in the system of the present invention, as described in Examples 3 and 6. Oxygen concentration in the system is measured by electrochemical microsensors applying a chronoamperometric protocol every minute. Lactate concentration is measured continuously. During the stop phase of pumping, cells consume oxygen, traced by the decrease in current (cf. Fig. 13). At the same time, lactate is produced and accumulates inside the chip (cf. Fig. 14). During the flow phase, the oxygen returns to the atmospheric baseline. The lactate signal shows a high increase at the beginning of the flow phase, as the lactate enriched-medium passes over the biosensor. As soon as fresh medium reaches the electrode, the signal drops again. The height of the peak is used as a measure for lactate production during culture. The microfluidic principle allows the quick determination of cellular consumption rates. Over days, an increase in cellular oxygen consumption can be observed as the organoids grow. Time intervals of stop/flow phases can be adjusted dynamically in order to compensate the increase in nutrient and oxygen demand of the cells over time. The addition of a drug (100 µM Antimycin A) suppresses the aerobic metabolism, as can be observed by reduced cellular consumption. Organoids maintain integrity and viability, as confirmed by microscopy.

The system of the present invention preferably allows for example the following:
- Directed supply of different organoid/cell compartments with at least two 3D cell types as well as concentration gradients resulting therefrom, which are to simulate a heterogeneous tumor.
- The technology used permits a gas-tight cell culture chamber in which the fluidics can be used to adjust the concentration of dissolved gas (e.g. setting of hypoxia).
- Continuous in situ monitoring of metabolic parameters oxygen, glucose and lactate in the 3D cell culture.
- Controllability of culture conditions and communication between the organoid/cell compartments.
- Small media volumes and thus rapid concentration changes of metabolites and reaction to stimuli (nutrients, active ingredients).
- Multiparametric electrochemical measurement of metabolites in-line (oxygen) and downstream (glucose, lactate).
- Sensor technology of metabolic parameters allows implicit flow measurement.
- Microscopability through the use of transparent materials (this also allows marker-based optical measurement techniques in the OCS).
- Possibility of continuous collection of smallest media samples for external analysis of biomarkers (metabolites, DNA, RNA, proteins).
- Handling, especially filling of the 3D cell cultures, comparable to the standard protocols for 3D cell cultures with Matrigel.
- Barrier structures allow bubble-free, targeted filling (separation of matrix gel vs. air) and compartmentalization during operation (separation of matrix gel vs. culture medium) without excluding diffusion for mass transport (metabolites, signal molecules).
- Scalability by adding further channels and compartments (application in drug screening).
- Fixation and removal of the cells after the experiment for further analysis.

## Claims

1. A system for monitoring three-dimensional cell cultures, wherein the system comprises
a substrate,
an isolation material disposed on the substrate and having openings, electrodes disposed in the openings of the isolation material, and
a fluidic unit disposed on the isolation material and having a first fluidic channel, a first cell compartment, a second fluidic channel, a second cell compartment, a third fluidic channel in this order along the surface of the isolation material, and barrier structures, which have openings, at each interface between the cell compartments and the fluidic channels;
wherein in at least one of the fluidic channels at least one working electrode is provided and
at least one reference electrode and at least one counter electrode are provided at a second end in fluid direction of at least one of the fluidic channels.

2. The system according to claim 1, wherein in at least one of the cell compartments at least one working electrode is provided.

3. The system according to claim 1 or 2, wherein at least one working electrode is provided in each of the fluidic channels.

4. The system according to any one of claims 1 to 3, wherein a reference electrode and a counter electrode are provided in the second fluidic channel.

5. The system according to any one of claims 1 to 4, wherein at least two working electrodes for monitoring oxygen are provided in each of the fluidic channels.

6. The system according to any one of claims 1 to 5, wherein a working electrode for monitoring glucose and/or a working electrode for monitoring lactate is provided at the second end in fluid direction of any fluidic channel.

7. The system according to claim 6, wherein the working electrode for monitoring glucose and/or the working electrode for monitoring lactate is/are covered with a hydrogel.

8. The system according to any one of claims 1 to 7, wherein the barrier structure comprises a continuous flat step, which preferably has a height of from 1.0 µm to 200 µm, and a plurality of posts provided on the step in equal distances along the fluid flow.

9. The system according to claim 8, wherein the distance between two adjacent posts is from 10 µm to 1.0 mm.

10. The system according to claim 8 or 9, wherein the posts have a triangular cross-sectional shape, with the triangles preferably having a flat side pointing to the side of the fluidic channels and a vertex pointing to the side of the cell compartments.

11. The system according to any one of claims 1 to 10, wherein the system further comprises contact pads, which are situated on the surface of the substrate outside the area where the fluidic unit is formed, and
circuit paths connecting individual electrodes to individual contact pads.

12. The system according to any one of claims 1 to 11, wherein the system further comprises a cover having openings corresponding to each of the cell compartments and the fluidic channels.

13. An organ-on-chip device comprising the system according any one of claims 1 to 12, an electrical interface to which the system is bonded, and cell cultures embedded in the cell compartments of the system.

14. The organ-on-chip device according to claim 13, wherein three-dimensional cell cultures embedded in a matrix are provided in the cell compartments of the system.

15. A method for monitoring cell cultures, wherein the method comprises the steps of
providing an organ-on-chip device according to claim 13 or 14,
introducing one or more fluids to one or more fluidic channels, and
detecting the signals of the electrodes.
